# EUROPEAN PATENT APPLICATION

(11) **EP 1 712 554 A1**
(43) Date of publication of application: **18.10.2006**
(21) Application number: 05709546.5
(22) Date of filing: 01.02.2005
(51) Int. Cl.: C07D 498/06, A23K 1/16, A23L 3/3544, A61K 31/5383, A61P 31/00, A61P 31/04, C07D 207/14

(54) **PYRIDOBENZOXAZINE DERIVATIVE**

(30) Priority: 02.02.2004 JP 2004025982
(71) Applicant: DAIICHI PHARMACEUTICAL CO., LTD., Chuo-ku, Tokyo 103-8234 (JP)
(72) Inventor: TAKEMURA, Makoto; c/o DAIICHI PHARMACEUTICAL CO, chome, Edogawa-ku, Tokyo; 1348630 (JP); OHKI, Hitoshi; c/o DAIICHI PHARMACEUTICAL CO.,, 1-chome, Edogawa-ku, Tokyo; 1348630 (JP)
(74) Representative: Reitstötter - Kinzebach
(86) International application number: PCT/JP2005/001400
(87) International publication number: WO 2005/073238

(57) **Abstract**

A quinolone antimicrobial agent having potent antimicrobial activity against gram positive and gram negative bacteria and having high safety is provided. A compound represented by the following formula (1): 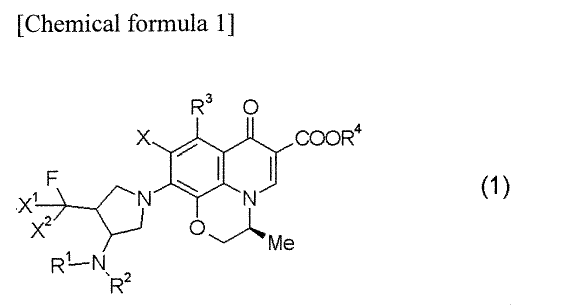 [wherein, R¹: hydrogen, alkyl, cycloalkyl, or substituted carbonyl derived from amino acid, peptide or tripeptide; R²: hydrogen, alkyl, or cycloalkyl; R³: hydrogen, amino, halogen, or alkyl; R⁴: hydrogen, phenyl, acetoxymethyl, pivaloyloxymethyl, ethoxycarbonyl, choline, dimethylaminoethyl, 5-indanyl, phthalidinyl, 5-alkyl-2-oxo-1,3-dioxol-4-ylmethyl, 3-acetoxy-2-oxobutyl, alkyl, alkoxymethyl, or phenylalkyl; X, X¹, X²: hydrogen or halogen] or salt thereof, or a hydrate thereof; and antimicrobial agent and therapeutic agent for infections, each containing the compound, salt thereof or a hydrate thereof.

## Description

### TECHNICAL FIELD

The present invention relates to quinolone-type synthetic antimicrobial agents useful as medicaments, veterinary drug, drug for fisheries, or antimicrobial preservatives.

### BACKGROUND ART

Since the discovery ofNorfloxacin, quinolone-type synthetic antimicrobial agents (including compounds having a pyridobenzoxazine nucleus) have improved antimicrobial activity and pharmacokinetics and therefore have grown to be chemotherapeutic agents effective for almost all systemic infections. Many such compounds are now provided for clinical purposes.
In recent years, bacteria having low sensitivity to quinolone-type synthetic antimicrobial agents have been increasing. For example, jusut like the gram-positive cocci such as methicillin-resistant *Staphylococcus aureus* (MRSA) and penicillin-resistant *Streptococcus pneumoniae* (PRSP) insensitive to β-lactam antibiotics, and vancomycin-resistant *Enterococci* (VRE) insensitive to aminoglycoside antimicrobial agents, gram-positive bacteria which have also acquired low insensitivity to quinolone synthetic antimicrobial agents in addition to having resistance against agents other than quinolone-type synthetic antimicrobial agents have been increasing. Accordingly, there still is a demand for the development of an agent having higher effectiveness particularly against gram positive cocci in the clinical field.
It has been revealed that some of quinolone-type synthetic antimicrobial agents have side effects of inducing convulsion when used in combination with a nonsteroidal anti-inflammatory agent, or side effects such as phototoxicity, hepatotoxicity, cardiac toxicity (abnormality causing life-threatening arrhythmia which is observed as an electrocardiographic abnormality) and abnormal blood sugar level so that there is also a demand for the development of quinolone-type synthetic antimicrobial agents having higher safety.
Levofloxacin (LVFX) is known as a representative drug of pyridobenzoxazine derivatives and has been widely used clinically as an antimicrobial chemotherapeutic agent having excellent safety. This Levofloxacin has such a structural characteristics having a 4-methylpiperazin-1-yl substituent at the 10-position of the pyridobenzoxazine mother nucleus.

It is known that the structure of a substituent at the 7-position (or 10-position in the case of a pyridobenzoxazine mother nucleus) of a quinolone nucleus has a great influence on the antimicrobial activity, pharmacokinetics and safety of quinolone-type synthetic antimicrobial agents. Among quinolone-type synthetic antimicrobial agents, quinolone derivatives having a 3-aminopyrrolidinyl group as the substituent at the 10-position of a pyridobenzoxazine mother nucleus are known to have stronger antimicrobial activity against gram negative and gram positive bacteria than quinolone derivatives having a piperazinyl group as the substituent (refer to Non-patent Documents 1 and 2).
Although the above-described quinolone derivatives having a 3-aminomethylpyrrolidin-1-yl group as the substituent exhibits strong antimicrobial activity, many of these compounds act on not only microorganisms but also eukaryotic cells owing to low selective toxicity (refer to Non-patent Document 2). A drug design having enhanced selective toxicity is therefore necessary when they are used as a medicament or veterinary drug. Appearance of a compound equipped with both potent antimicrobial activity and high selective toxicity is therefore requested clinically.

Pyridobenzoxazine derivatives having a 3-amino-4-(alkyl-substituted pyrrolidin)-1-yl group as the substituent at the 7-position, quinolone-carboxylic acid derivatives (A) having a cis-3-amino-4-methylpyrrolidin-1-yl group as the substituent at the 10-position of the pyridobenzoxazine mother nucleus are for example described in Patent Document 1. The document does not include, however, a specific description of pyridobenzoxazine derivatives having a cis-3-amino-4-fluorine-substituted-methylpyrrolidin-1-yl group as the substituent at the 10-position.

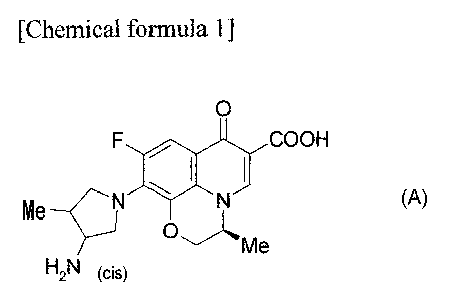

Quinolone-carboxylic acid derivatives (B) having a cis-3-amino-4-fluoromethylpyrrolidin-1-yl group as the substituent at the 7-position are disclosed in Patent Document 2 and Non-patent document 3 (the definition of the substituent in the formula (B) is according to Patent Document 2 and has no relation with the definition of the substituent in this specification even if it is indicated by the same reference numerals).

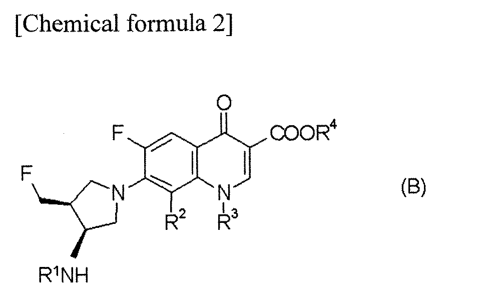

The substituent at the 8-position of this quinolone nucleus is limited to an alkoxy group having from 1 to 6 carbon atoms typified by a methoxy group or a halogenomethoxy group.
No description is included on derivatives having united substituents at the 1-position and 8-position of the quinolone nucleus each other. In addition, Patent Document 2 includes no specific description on compounds having a cis-3-amino-4-(fluorine-substituted-methyl-pyrrolidin)-1-yl group as the substituent at the 10-position of the mother nucleus.

In Non-patent Document 4, quinolone-carboxylic acid derivatives having, as the substituent at the 7-position thereof, a cis-3-amino-4-fluoro-substituted-methylpyrrolidin-1-yl group are disclosed. As a specific example of them, 8-methoxyquinolone derivatives (C) having, as the substituent thereof, a cis-3-amino-4-trifluoromethylpyrrolidin-1-yl group are described. However, the compounds described in Non-patent Document 4 are only quinolone-carboxylic acid derivatives and no specific description of pyridobenzoxazines having a cis-3-amino-4-(fluorine-substituted-methyl-pyrrolidin)-1-yl group as the substituent at the 10-position of the mother nucleus is included.

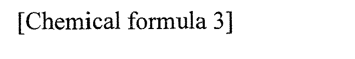

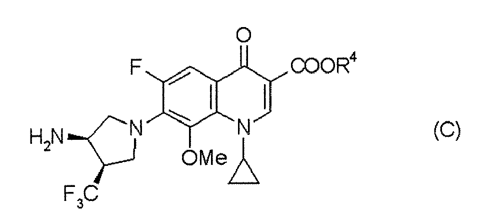

In Patent Document 2 and Non-patent Documents 3 and 4, cis-3-amino-4-fluorine-substituted pyrrolidine derivatives are exemplified, but they do not include any description on quinolone derivatives having a cis-3-amino-4-difluoropyrodine substituent which are embraced in the present invention. In addition, they include neither specific description nor examples of a production process of cis-3-amino-4-difluoromethylpyrrolidine derivatives and intermediates for the production thereof relating to the present invention.
Patent Document 1: European Patent No. 208291 (JP-A-62-4264)
Patent Document 2: International Patent Publication No. 98-58923
Non-patent Document 1: International Journal of Antimicrobial Agents, Vol. 16, p. 5 (2000)
Non-patent Document 2: Journal of Antimicrobial Chemotherapy, Vol. 33, p. 685(1994)
Non-patent Document 3: Chemical Pharmaceutical Bulletin, Vol. 48 (No. 11), p. 1667 (2000)
Non-patent Document 4: Bioorganic Medicinal Chemistry Letters, Vol. 8, p. 2833 (1998)

### DISCLOSURE OF THE INVENTION

### PROBLEMS THAT THE INVENTION IS TO SOLVE

Accordingly, an object of the present invention is to provide a quinolone antimicrobial agent and a therapeutic agent for infections exhibiting potent antimicrobial activity against gram positive and gram negative bacteria and at the same time having high safety.

### MEANS FOR SOLVING THE PROBLEMS

The present inventors found that compounds represented by the formula (1) of the present invention exhibit broad-spectrum and potent antimicrobial activity against gram positive and gram negative bacteria, especially against resistant bacteria typified by resistant gram-positive cocci including MRSA and PRSP and moreover they can be used safely as an antimicrobial medicament for human use, thus accomplishing the present invention.
The present invention relates to a compound represented by the following formula (1):

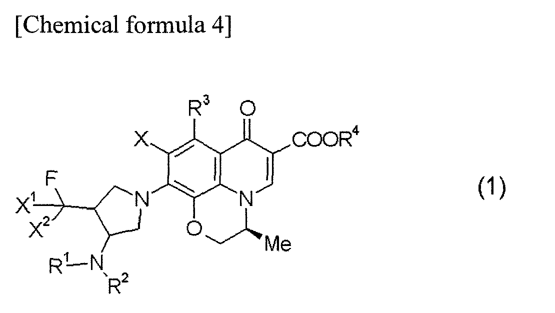

[wherein, R¹ represents a hydrogen atom, an alkyl group having from 1 to 6 carbon atoms or a cycloalkyl group having from 3 to 6 carbon atoms, or a substituted carbonyl group derived from an amino acid, dipeptide or tripeptide, with the proviso that the alkyl group may have, as the substituent, at least one group selected from the group consisting of a hydroxyl group, an amino group, halogen atoms, alkylthio groups having from 1 to 6 carbon atoms, and alkoxy groups having from 1 to 6 carbon atoms;
R² represents a hydrogen atom, an alkyl group having from 1 to 6 carbon atoms or a cycloalkyl group having from 3 to 6 carbon atoms, with the proviso that the alkyl group may have, as the substituent, at least one group selected from the group consisting of a hydroxyl group, an amino group, halogen atoms, alkylthio groups having from 1 to 6 carbon atoms, and alkoxy groups having from 1 to 6 carbon atoms;
R³ represents a hydrogen atom, amino group, halogen atom or alkyl group having from 1 to 6 carbon atoms, with the proviso that the amino group may have, as the substituent, one or two groups selected from the group consisting of a formyl group, alkyl groups having from 1 to 6 carbon atoms and acyl groups having from 2 to 6 carbon atoms;
R⁴ represents a hydrogen atom, phenyl group, acetoxymethyl group, pivaloyloxymethyl group, ethoxycarbonyl group, choline group, dimethylaminoethyl group, 5-indanyl group, phthalidinyl group, 5-alkyl-2-oxo-1,3-dioxol-4-ylmethyl group, 3-acetoxy-2-oxobutyl group, alkyl group having from 1 to 6 carbon atoms, alkoxymethyl group having from 2 to 7 carbon atoms, or a phenylalkyl group composed of an alkylene group having from 1 to 6 carbon atoms and a phenyl group;
X¹ and X² each independently represents a hydrogen atom or halogen atom, and
X represents a hydrogen atom or a halogen atom], salt thereof, or a hydrate thereof.
The present invention also relates to:
the above-described compound, salt thereof, or hydrate thereof, wherein the compound of the formula (1) is a stereochemically pure compound;
the above-described compound, salt thereof, or hydrate thereof, wherein the compound of the formula (1) is a compound having a structure represented by the following formula:

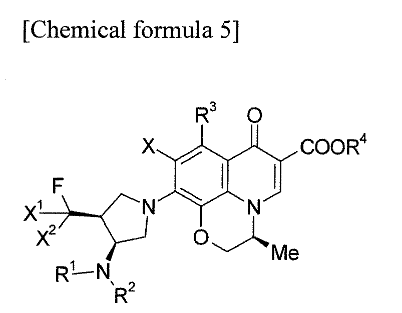

[wherein, R¹, R², R³, R⁴, X¹, X² and X have the same definitions as described above];
the above-described compound, salt thereof, or hydrate thereof, wherein in the formula (1), X¹ and X² each represents a hydrogen atom;
the above-described compound, salt thereof, or hydrate thereof, wherein in the formula (1), either one of X¹ or X² represents a fluorine atom, while the other one represents a hydrogen atom;
the above-described compound, salt thereof, or hydrate thereof, wherein in the formula (1), R¹ and R² each represents a hydrogen atom;
the above-described compound, salt thereof, or hydrate thereof, wherein in the formula (1), R¹ represents a substituted carbonyl group derived from an amino acid, dipeptide or tripeptide and R² represents a hydrogen atom;
the above-described compound, salt thereof, or hydrate thereof, wherein in the formula (1), either one of R¹ or R² represents a hydrogen atom and the other one is a cyclopropyl group;
the above-described compound, salt thereof, or hydrate thereof, wherein in the formula (1), X represents a fluorine atom,
the above-described compound, salt thereof, or hydrate thereof, wherein in the formula (1), R³ represents a hydrogen atom;
the above-described compound, salt thereof, or hydrate thereof, wherein in the formula (1), R⁴ represents a hydrogen atom;
10-[3-(S)-amino-4-(S)-fluoromethylpyrrolidin-1-yl]-9-fluoro-2,3-dihydro-3-(S)-methyl-7-oxo-7H-pyrido[1,2,3-de][1,4]benzoxazine-6-carboxylic acid, salt thereof, or a hydrate thereof;
10-[3-(S)-amino-4-(S)-difluoromethylpyrrolidin-1-yl]-9-fluoro-2,3-dihydro-3-(S)-methyl-7-oxo-7H-pyrido[1,2,3-de][1,4]benzaxazine-6-carboxylic acid, salt thereof, or a hydrate thereof;
a medicament comprising the above-described compound, salt thereof, or a hydrate thereof as an effective ingredient;
an antimicrobial agent or antimicrobial preparation comprising the above-described compound, salt thereof, or a hydrate thereof as an effective ingredient;
a therapeutic agent or therapeutic preparation for infections, which comprises the above-described compound, salt thereof, or a hydrate thereof as an effective ingredient;
a method of treating a disease, which comprises administering an effective amount of the above-described compound, salt thereof, or a hydrate thereof;
a method of treating an infection, which comprises administering an effective amount of the above-described compound, salt thereof, or a hydrate thereof;
a process of producing a medicament, which comprises incorporating the above-described compound, salt thereof, or a hydrate thereof as an effective ingredient;
a process of producing an antimicrobial agent, which comprises incorporating the above-described compound, salt thereof, or a hydrate thereof as an effective ingredient;
a process of producing a therapeutic agent for an infection, which comprises incorporating the above-described compound, salt thereof, or a hydrate thereof as an effective ingredient; and the like.

The present invention also relates to a compound represented by the following formula (3):

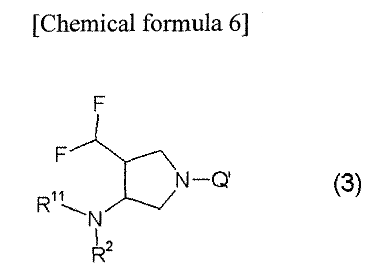

[wherein, R¹¹ represents a hydrogen atom, an alkyl group having from 1 to 6 carbon atoms or a cycloalkyl group having from 3 to 6 carbon atoms, or an amino-protecting group, or a substituted carbonyl group derived from an amino acid, dipeptide or peptide having an amino group which is unsubstituted or may be protected, with the proviso that the alkyl group may have, as the substituent, at least one group selected from the group consisting of a hydroxyl group, an amino group, halogen atoms, alkylthio groups having from 1 to 6 carbon atoms and alkoxy groups having from 1 to 6 carbon atoms;
R² has the same meaning as defined in the formula (1); and
Q' means an amino-protecting group or hydrogen atom], salt thereof, or a hydrate thereof;
or the above-described compound, salt thereof, or hydrate thereof, wherein the compound of the formula (3) has a structure represented by the following formula:

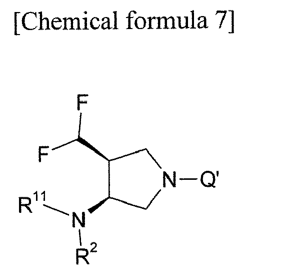

[wherein, R¹¹, R² and Q' each has the same meaning as defined above].
The present invention further relates to:
the above-described compound, salt thereof, or hydrate thereof, wherein the amino-protecting group is selected from the group consisting of alkoxycarbonyl groups which may have a substituent, aralkyloxycarbonyl groups which may have a substituent, acyl groups which may have a substituent, alkyl groups which may have a substituent, aralkyl groups which may have a substituent, and substituted silyl groups;
the above-described compound, salt thereof, or hydrate thereof, wherein the amino-protecting group is selected from the group consisting of tertiary butoxycarbonyl group, 2,2,2-trichloroethoxycarbonyl group, benzyloxycarbonyl group, para-methoxybenzyloxycarbonyl group, para-nitrobenzyloxycabonyl group, acetyl group, methoxyacetyl group, trifluoroacetyl group, chloroacetyl group, pivaloyl group, formyl group, benzoyl group, tertiary butyl group, benzyl group, paranitrobenzyl group, para-methoxybenzyl group, (R)-1-phenylethyl group, (S)-1-phenylethyl group, triphenylmethyl group, methoxymethyl group, tertiary butoxymethyl group, tetrahydropyranyl group, 2,2,2-trichloroethoxymethyl group, trimethylsilyl group, isopropyldimethylsilyl group, tertiary butyldimethylsilyl group, tribenzylsilyl group, and tertiary butyldiphenylsilyl group;
the above-described compound, salt thereof, or hydrate thereof, wherein R¹¹ and Q' represent amino-protecting groups which are not the same;
the above-described compound, salt thereof, or hydrate thereof, wherein R¹¹ and Q' represent amino-protecting groups which are deprotected or cleaved under different reaction conditions;
the above-described compound, salt thereof, or hydrate thereof, wherein the compound of the formula (3) is a stereochemically pure compound;
3-(S)-(tert-butoxycarbonylamino)-4-(S)-difluoromethyl-1-(R)-phenylmethylpyrrolidine, salt thereof, or a hydrate thereof ;
3-(S)-(tert-butoxycarbonylamino)-4-(S)-difluoromethyl-pyrrolidine, salt thereof, or a hydrate thereof;
use of the above-described compound, salt thereof, or hydrate thereof for the production of the compound represented by the formula (1), and the like.

### EFFECTS OF THE INVENTION

The pyridobenzoxazine derivatives according to the present invention have particularly excellent antimicrobial activity against gram positive and gram negative bacteria. The pyridobenzoxazine derivatives of the present invention are therefore useful as an antimicrobial agent and a therapeutic agent for infections. In addition, the pyridobenzoxazine derivatives of the invention show week acute toxicity and higher safety.

### BEST MODE FOR CARRYING OUT THE INVENTION

Each of the substituents of the compounds represented by the following formula (1):

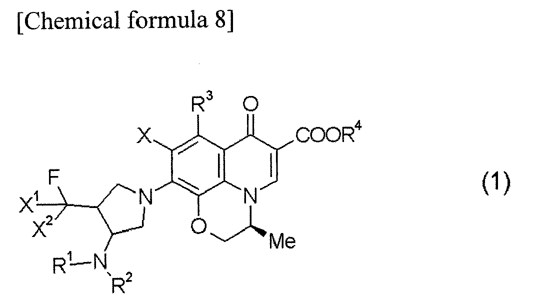

[R¹, R², R³, R⁴, X¹, X² and X have the same definitions as defined above] will next be described.

The substituent R¹ represents a hydrogen atom, an alkyl group having from 1 to 6 carbon atoms, or a cycloalkyl group having from 3 to 6 carbon atoms, or a substituted carbonyl group derived from an amino acid, dipeptide or tripeptide. The substituent R² represents a hydrogen atom, an alkyl group having from 1 to 6 carbon atoms or a cycloalkyl group having from 3 to 6 carbon atoms.
When R¹ or R² represents an alkyl group, it may have, as the substituent, at least one group selected from the group consisting of a hydroxyl group, an amino group, halogen atoms, alkylthio groups having from 1 to 6 carbon atoms, and alkoxy groups having form 1 to 6 carbon atoms.

When R¹ or R² represents an alkyl group, the alkyl group may be either linear or branched. It is preferably a methyl group, ethyl group, propyl group or isopropyl group, of which methyl and ethyl groups are more preferred and methyl group is still more preferred.
When the alkyl group has a hydroxyl or amino group as its substituent, they may preferably be located on the terminal carbon atom of the alkyl group. The alkyl group having a hydroxyl group preferably has carbon atoms up to three and hydroxymethyl, 2-hydroxyethyl, 2-hydroxypropyl and 3-hydroxypropyl groups are preferred. The alkyl group having an amino group preferably has carbon atoms up to three and aminomethyl, 2-aminoethyl, 2-aminopropyl and 3-aminopropyl groups are preferred.
When the alkyl group has a halogen atom as its substituent, the alkyl group may be either linear or branched one having from 1 to 6 carbon atoms. A fluorine atom is preferred as the halogen atom. With regard to the number of fluorine atoms, any one of from mono-substituted to perfluoro-substituted ones may be employed. Examples include monofluoromethyl, difluoromethyl, trifluormethyl and 2,2,2-trifluoroethyl groups.

When the alkyl group has an alkylthio or alkoxy group as the substituent, the alkyl group may either linear or branched. In the alkylthio or alkoxy group, the alkyl group may be either linear or branched. Preferred examples of the alkyl group having an alkylthio group include alkylthiomethyl, alkylthioethyl and alkylthipropyl in which alkylthio group having from 1 to 3 carbon atoms is preferred. More preferred examples include methylthiomethyl, ethylthiomethyl and methylthioethyl groups. Preferred examples of the alkyl group having an alkoxy group include alkoxymethyl, alkoxyethyl and alkoxypropyl groups, in which the alkoxy group having from 1 to 3 carbon atoms is preferred. More preferred examples include methoxymethyl, ethoxymethyl and methoxyethyl groups.

When R¹ or R² represents a cycloalkyl group, the cycloalkyl group is preferably a cyclopropyl or cyclobutyl group, with a cyclopropyl group being more preferred.
As a combination of R¹ and R², that of R¹ representing a hydrogen atom, an alkyl group, a cycloalkyl group, or a substituted carbonyl group derived from an amino acid, dipeptide or tripeptide and R² representing a hydrogen atom is preferred. A combination of R¹ representing a hydrogen atom, an alkyl group or a cycloalkyl group and R² representing a hydrogen atom is more preferred. The alkyl group is preferably a methyl or ethyl group, especially preferably a methyl group. The cycloalkyl group is preferably a cyclopropyl or cyclobutyl group, especially preferably a cyclopropyl group. As the combination of R¹ and R², that of R¹ and R² each representing a hydrogen atom or that of R¹ representing a methyl group and R² representing a hydrogen atom is still more preferred.
A quinolone derivative having, as the substituent R¹, a substituted carbonyl group derived from an amino acid, dipeptide or tripeptide and, as R², a hydrogen atom is particularly useful as a prodrug. A specific example relating to these will be described later.

The substituent R³ represents a hydrogen atom, an amino group, a halogen atom or an alkyl group having from 1 to 6 carbon atoms. The amino group may have, as the substituent, one or two groups selected from the group consisting of formyl group, alkyl groups having from 1 to 6 carbon atoms, and acyl groups having from 2 to 6 carbon atoms.
When R³ represents an alkyl group, it may be either a linear or branched group having from 1 to 6 carbon atoms and is preferably a methyl, ethyl, propyl or isopropyl group. Of these, methyl and ethyl groups are preferred, of which a methyl group is more preferred.
The substituent R³ is preferably a hydrogen atom, an alkyl group or an amino group, of which a hydrogen atom, a methyl group or an unsubstituted amino group (-NH₂) is especially preferred, with a hydrogen atom being still more preferred.

The substituent R⁴ represents a hydrogen atom, a phenyl group, an acetoxymethyl group, a pivaloyloxymethyl group, an ethoxycarbonyl group, a choline group, a dimethylaminoethyl group, a 5-indanyl group, a phthalidinyl group, a 5-alkyl-2-oxo-1,3-dioxol-4-ylmethyl group, a 3-acetoxy-2-oxobutyl group, an alkyl group having from 1 to 6 carbon atoms, an alkoxymethyl group having from 2 to 7 carbon atoms, or a phenylalkyl group composed of an alkylene group having from 1 to 6 carbon atoms and a phenyl group.

When the compound of the present invention is used for an antimicrobial purpose, use of a carboxylic acid compound having a hydrogen atom as the substituent R⁴ is preferred. A quinolone derivative having a carboxylic acid moiety converted to an ester is useful as a synthetic intermediate or prodrug, which will be described later.

The substituents X¹ and X² each independently represents a hydrogen atom or a halogen atom. As the halogen atom, a fluorine atom is especially preferred.
As the combination of X¹ and X², that of X¹ and X² each representing a hydrogen atom and that of X¹ representing either one of a hydrogen atom or a fluorine atom and X² representing the other one is preferred.

The substituent X represents a hydrogen atom or halogen atom. As the halogen atom, a fluorine atom is especially preferred. As X, a fluorine atom is more preferred.

The compound of the present invention exhibits excellent characteristics based on having a group of a structure represented by the following formula (5):

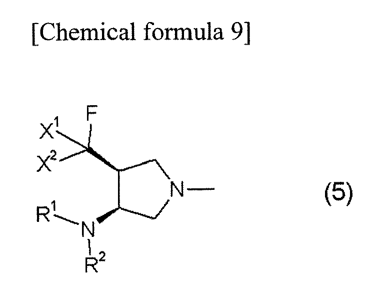

at the 10-position of the 9-fluoro-2,3-dihydro-3-(S)-methyl-7-oxo-7H-pyrido[1,2,3-de][1,4]benzoxazine-6-carboxylic acid nucleus and moreover a fluorine atom substituted at the 9-position of the nucleus.
The substituent represented by the formula (5) is characterized in that the amino substituent at the 3-position and the fluoromethyl group at the 4-position on the pyrrolidine ring are in a cis configuration. The cis isomer preferably has an absolute configuration of (3S,4S).

It has been revealed that since the compound of the present invention has, as the substituent at the 10-position of the 9-fluoro-2,3-dihydro-3-(S)-methyl-7-oxo-7H-pyrido[1,2,3-de][1,4]benzoxazine-6-carboxylic acid nucleus, a 3-(S)-amino-4-(S)-fluoromethylpyrrolidin-1-yl or 3-(S)-amino-4-(S)-difluoromethylpyrrolidin-1-yl group and has a fluorine atom substituted at the 9-position of the nucleus, it exhibits excellent characteristics such as antimicrobial activity, pharmacokinetics and safety.

When the compound of the formula (1) according to the present invention has a structure permitting existence of diastereomers, administration of the invention compound composed of a pure diastereomer is preferred when it is administered to humans or animals. The term "the invention compound composed of a pure diastereomer" as used herein embraces not only the case where the invention compound does not contain another diastereomer at all but also the case where the invention compound is chemically pure.
In other words, the invention compound may contain another diastereomer insofar as it does not have an influence on the physical constant or physiological activity of the invention compound.

The term "the invention compound composed of a pure diastereomer" also means that when asymmetric carbon atoms contained in the compound permit the existence of several isomers, the invention compound is composed of one of the several isomers. The above definition of the term "pure" equally applies to this case.

The 9-fluoro-2,3-dihydro-3-(S)-methyl-7-oxo-7H-pyrido[1,2,3-de][1,4]benzoxazine-6-carboxylic acid derivative of the present invention may be in free form, but may be converted into an acid addition salt or a salt of a carboxy group. Examples of the acid addition salt include inorganic acid salts such as hydrochloride, sulfate, nitrate, hydrobromide, hydroiodide and phosphate; and organic acid salts such as methanesulfonate, benzenesulfonate and toluenesulfonate (sulfonates) and acetate, citrate, maleate, fumarate and lactate (carboxylates).

Examples of the salts of a carboxy group include alkali metal salts such as lithium salt, sodium salt and potassium salt, alkaline earth metal salts such as magnesium salt and calcium salt, and ammonium salts, triethylamine salts, N-methylglucamine salts, and tris-(hydroxymethylhydroxymethylaminomethane salts. Of these, either inorganic acid salts or organic acid salts may be used.

The 9-fluoro-2,3-dihydro-3-(S)-methyl-7-oxo-7H-pyrido[1,2,3-de][1,4]benzoxazine-6-carboxylic acid derivative in free form, or in the form of an acid addition salt or salt of a carboxy group may be present as a hydrate.

When the compound of the present invention is used for an antimicrobial purpose, a carboxylic acid compound having a hydrogen atom as the substituent R⁴ is preferred. A pyridobenzoxazine derivative having a carboxylic acid moiety converted into an ester is, on the other hand, useful as a synthetic intermediate or prodrug. For example, its alkyl esters, benzyl esters, alkoxyalkyl esters, phenylalkyl esters and phenyl esters are useful as a synthetic intermediate.

The ester to be used as a prodrug is an ester which is easily cleaved in vivo and forms a carboxylic acid compound in free form. Examples of those include acetoxymethyl ester, pivaloyloxymethyl ester, ethoxycarbonyl ester, choline ester, dimethylaminoethyl ester, 5-indanyl ester, phthalidinyl ester, and oxoalkyl esters such as 5-alkyl-2-oxo-1,3-dioxol-4-ylmethyl ester and 3-acetoxy-2-oxobutyl ester.

Pyridobenzoxazine derivatives having as the substituent R¹ a substituted carbonyl group derived from an amino acid, dipeptide or tripeptide and as R² a hydrogen atom are also useful as a prodrug.

The amino acid, dipeptide or tripeptide to be used for obtaining such a prodrug forms a free amine compound by the in vivo cleavage of a peptide bond formed by the carboxy group of the amino acid, dipeptide or tripeptide and amino group present in the substituent at the 10-position of the pyridobenzoxazine derivative. Examples of a substituent for obtaining such a prodrug include substituted carbonyl groups derived from amino acids such as glycine, alanine and aspartic acid, dipeptides such as glycine-glycine, glycine-alanine, and alanine-alanine, and tripeptides such as glycine-glycine-alanine and glycine-alanine-alanine.

The compound of the present invention represented by the formula (1) can be prepared in various manners. As one preferred example, the compound can be prepared, for example, by reacting a compound represented by the following formula (6):

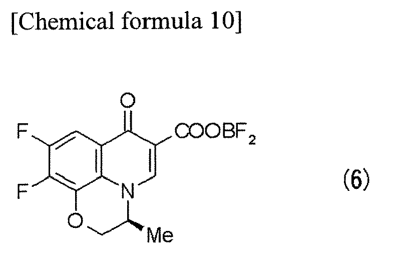

with a compound represented by the following formula (7):

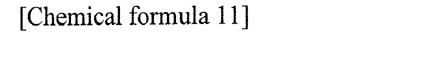

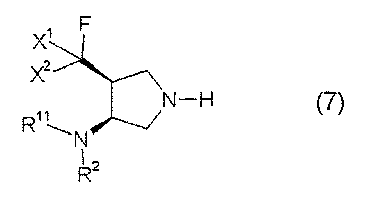

[wherein, R¹¹ represents a hydrogen atom, an alkyl group having from 1 to 6 carbon atoms or a cycloalkyl group having from 3 to 6 carbon atoms, an amino-protecting group, or a carbonyl group derived from an amino acid, dipeptide or tripeptide having an amino group which is unsubstituted or protected with an amino-protecting group, with the proviso that the alkyl group may have, as the substituent, a group selected from the group constituting of a hydroxyl group and an amino group which may be protected, halogen atoms, alkylthio groups having from 1 to 6 carbon atoms and alkoxy groups having from 1 to 6 carbon atoms,
R² has the same meaning as defined above in (1), and
X¹ and X² each independently represents a hydrogen atom or halogen atom] or acid addition salt thereof (in the case of an addition salt, the reaction is performed in the presence of a reagent for converting the salt into free form).
Examples of the acid addition salt include inorganic acid salts such as hydrochloride, sulfate, nitrate, hydrobromide, hydroiodide and phosphate, and organic acid salts such as methanesulfonate, benzenesulfonate and toluenesulfonate (sulfonates) and acetate, citrate, maleate, fumarate and lactate (carboxylates).

The reaction can be conducted with or without a solvent. Any solvent is usable in the reaction insofar as it does not adversely affect the reaction. Examples include dimethylsulfoxide, pyridine, acetonitrile, ethanol, chloroform, dimethylformamide, dimethylacetamide, N-methylpyrrolidone, tetrahydrofuran, water and 2-methoxybutanol and mixtures thereof.

The reaction is preferably performed in the presence of an acid acceptor such as an inorganic base or organic base, for example, an inorganic basic compound such as a carbonate or hydrogen carbonate of an alkali metal or alkaline earth metal, or an organic basic compound such as triethylamine, pyridine, 1,8-diazabicycloundecene, N-methylpiperidine or N,N-diisopropylethylamine.

The reaction temperature is usually within a range of from room temperature to 200°C, preferably from 25 to 150°C. The reaction time may fall within a range of from 30 minutes to 48 hours. The reaction is usually completed within from about 30 minutes to 8 hours.

Relative to the compound represented by the formula (6), preferably from 0.5 to 3.0 equivalents, more preferably from 1.0 to 2.0 equivalents, still more preferably from 1.0 to 1.2 equivalents of the compound represented by the formula (7) is added in the presence of the basic compound.

When the substituent R¹¹ is not an amino-protecting group and R² has the same meaning as described in the formula (1), for example, when R¹¹ is an alkyl group and moreover, this alkyl group has a hydroxyl or amino group as the substituent, the hydroxyl or amino group is unsubstituted or may be protected. When R¹¹ is a substituted carbonyl group derived from an amino acid, dipeptide or tripeptide, the amino group thereof is preferably protected by a protecting group. The protecting group to be used for this purpose may be similar to that of R¹¹. The substituents X¹ and X² have the same meanings as described in the formula (1).

As the amino-protecting group, any protecting group usually employed in this field may be used. Examples include alkoxycarbonyl groups which may have a substituent such as tertiary butoxycarbonyl group and 2,2,2-trichloroethoxycarbonyl group; aralkyloxycarbonyl groups which may have a substituent such as benzyloxycarbonyl group, para-methoxybenzyloxycarbonyl group and paranitrobenzyloxycarbonyl group; acyl groups which may have a substituent such as acetyl group, methoxyacetyl group, trifluoroacetyl group, chloroacetyl group, pivaloyl group, formyl group and benzoyl group; alkyl groups which may have a substituent or aralkyl groups which may have a substituent such as tertiary butyl group, benzyl group, paranitrobenzyl group, para-methoxybenzyl group and triphenylmethyl group; ethers which may have a substituent such as methoxymethyl group, tertiary butoxymethyl group, tetrahydropyranyl group and 2,2,2-trichloroethoxymethyl group; and substituted silyl groups such as trimethylsilyl group, isopropyldimethylsilyl group, tertiary butyldimethylsilyl group, tribenzylsilyl group and tertiary butyldiphenylsilyl group.

When the deprotection is required, the target compound represented by the formula (1) is obtained by removing the protecting group under appropriately selected reaction conditions suited therefor.
The compound represented by the formula (7) can also be obtained by removing Q' from a compound represented by the following formula (8):

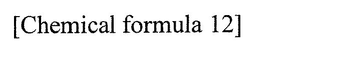

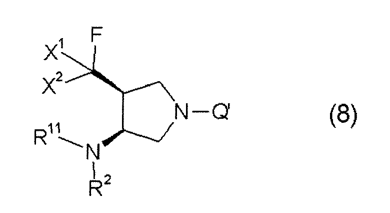

[wherein, R¹¹, R², X¹ and X² have the same meanings as defined in the formula (7); and
Q' represents an amino-protecting group, with the proviso that the amino protecting group may be selected from the group consisting of alkoxycarbonyl groups which may have a substituent, aralkyloxycarbonyl groups which may have a substituent, acyl groups which may have a substituent, alkyl groups which may have a substituent, aralkyl groups which may have a substituent and substituted silyl groups].

The above-described compound may be present as a salt, hydrate or a hydrate of a salt. Examples of an acid addition salt include inorganic acid salts and organic acid salts. Specific examples of them include inorganic acid salts such as hydrochloride, sulfate, nitrate, hydrobromide, hydroiodide and phosphate; and organic acid salts such as methanesulfonate, benzenesulfonate and toluenesulfonate (sulfonates) and acetate, citrate, maleate, fumarate and lactate (carboxylates).

When R¹¹ represents a substituted carbonyl group derived from an amino acid, dipeptide or tripeptide, it may be introduced in the stage of the formula (7) or formula (8), or it may be introduced after obtaining a compound of the formula (1) having as R¹ and R² a hydrogen atom.

When R¹¹ and Q' are each an amino-protecting groups, they may be the same or different but they may be removed under reaction conditions which are different from each other. Described specifically, it is convenient for the preparation of the compound (1) that one of them is removed selectively but the other one is a protecting group which remains unremoved.
As examples of the R¹¹ and Q' which are amino-protecting groups, following groups can be given. They are alkoxycarbonyl groups which may have a substituent, alralkyloxycarbonyl groups which may have a substituent, acyl groups which may have a substituent, alkyl groups which may have a substituent, aralkyl groups which may have a substituent and substituted silyl groups.

Specific examples include alkoxycarbonyl groups which may have a substituent such as tertiary butoxycarbonyl group and 2,2,2-trichloroethoxycarbonyl group; aralkyloxycarbonyl groups which may have a substituent such as benzyloxycarbonyl group, para-methoxybenzyloxycarbonyl group and paranitrobenzyloxycarbonyl group; acyl groups which may have a substituent such as acetyl group, methoxyacetyl group, trifluoroacetyl group, chloroacetyl group, pivaloyl group, formyl group and benzoyl group; alkyl groups which may have a substituent or aralkyl groups which may have a substituent such as tertiary butyl group, benzyl group, paranitrobenzyl group, para-methoxybenzyl group and triphenylmethyl group; ethers such as methoxymethyl group, tertiary butoxymethyl group, tetrahydropyranyl group and 2,2,2-trichloroethoxymethyl group; and substituted silyl groups such as trimethylsilyl group, isopropyldimethylsilyl group, tertiary butyldimethylsilyl group, tribenzylsilyl group and tertiary butyldiphenylsilyl group.

For the preparation of the compound of the formula (1), a B(OAc)₂ chelate compound may be used instead of the BF₂ chelate compound represented by the formula (6). Alternatively, such a boron chelate compound may be replaced by a COOH compound.

The compound of the present invention has potent antimicrobial activity so that it can be used as medicaments for human, animals and fisheries, agrichemicals or food preservatives.

When the invention compound is used as a medicament for human, its dosage differs depending on the age or weight of a patient to be administered, kind of bacterium which has caused infection, and degree of infection. The daily dosage may fall within a range of from 50 mg to 1 g, preferably from 100 mg to 500 mg, per adult.
When it is administered to an animal, the dosage differs depending on the purpose of administration (treatment or prevention), kind or size of the animal to be administered, kind of bacterium which has caused infection, and degree of infection. The daily dosage may usually fall within a range of from 1 mg to 200 mg, preferably from 5 mg to 100 mg per kg of the animal.
The daily dosage may be administered once or in two to four portions a day. The daily dosage may exceed the above-described amount if necessary.

The invention compound is active to a broad spectrum of microorganisms causing various infections and it can treat, prevent or alleviate diseases caused by these pathogenic bacteria.

Examples of the bacteria or bacteria-like microorganisms against which the invention compound is effective include *Staphylococcus, Streptococcus pyogenes, Streptococcus hemolyticus, enterococci, pneumococci, Peptostreptococcus, gonococci, Escherichia coli, Citrobacter, Shigella, Klebsiella pneumoniae, Enterobacter, Serratia, Proteus, Pseudomonas aeruginosa, Haemophilus influenzae, Acinetobacter, Campylobacter,* and *Chlamydia trachomatis.*

Examples of diseases caused by such pathogens include folliculitis, furuncle, carbuncle, erysipelas, cellulitis, lymphangitis (lymphadenitis), felon, subcutaneous abscess, hidrosadenitis, acne conglobata, infectious atheroma, perirectal abscess, mastitis, superficial secondary infections of trauma, burn, surgical wound or the like, pharyngolaryngitis, acute bronchitis, tonsillitis, chronic bronchitis, bronchiectasis, diffuse pan-bronchiolitis, secondary infection of chronic respiratory diseases, pneumonia, pyelonephritis, cystitis, prostatitis, epididymis, gonococcal urethritis, nongonococcal urethritis, cholecystitis, cholangitis, shigellosis, enteritis, uterine adnexitis, intrauterine infection, bartholinitis, blepharitis, hordeolum, dacryocystitis, inflammation of the tarsal gland, corneal ulcer, otitis media, sinusitis, periodontitis, pericoronitis, jaw inflammation, peritonitis, endocarditis, sepsis, meningitis, and skin infections.

Examples of mycobacteria against which the invention compound is effective include tuberculosis complex [*Mycobacterium* (which will hereinafter be abbreviated as *"M"*) *tuberculosis, M bovis, M africanum*] and atypical mycobacteria [*M. kansasii, M marinum, M. scrofulaceum, M. avium, M. intracellularae, M xenopi,* and *M. fortuitum,* and *M chelonae*].

Mycobacterial infections caused by such pathogens can be classified roughly into three groups such as tuberculosis, atypical mycobacteriosis and leprosy depending on causative bacteria. Tuberculosis infection appears not only in lungs but also in chest cavity, trachea·bronchus, lymph node, generalized dissemination, bone and joint, meninx·brain, digestive organs (intestines-liver), skin, mammary gland, eye, middle ear-pharynx, urinary tract, male genitals, and female genitals. The main organ affected by atypical mycobacteria (non-tuberculosis mycobacteria) is a lung and additional examples include regional lymphadenitis, skin soft tissue, bone and joint, and systemic disseminated type.
The invention compound is also effective against various microorganisms causative of animal infections such as *Escherichia, Salmonella, Pasteurella, Haemophilus, Bordetella, Staphylococcus,* and Mycoplasma.

Specific examples of the disease include avian diseases such as colibacillosis, pullorum disease, avian paratyphoid, avian cholera, infectious coryza, staphylococcal infection, and mycoplasmosis; swine diseases such as colibacillosis, salmonellosis, pasteurellosis, hemophilosis, atrophic rhinitis, exudative epidermitis, and mycoplasmosis; bovine diseases such as colibacillosis, salmonellosis, hemorrhagic sepsis, mycoplasmosis, contagious pleuropneumonia of cattle, and mastitis; canine diseases such as *Escherichia coli* sepsis, salmonellosis, hemorrhagic sepsis, pyometra, and cystitis; and feline diseases such as exudative pleurisy, cystitis, chronic rhinitis, hemophilosis, kitten diarrhea and mycoplasmosis.

The antimicrobial preparation containing the invention compound can be obtained by selecting a proper dosage form suited for the administration route and employing a process usually employed for the corresponding dosage form. Examples of the dosage form of the antimicrobial preparation containing the invention compound include oral preparations such as tablets, powders, granules, capsules, liquids and solutions, syrups, elixirs, and oily or water-based suspensions. The antimicrobial preparation may contain a pharmaceutically acceptable carrier or diluent.
The preparation can be obtained by a known formulation technology and proper pharmaceutically acceptable additives can be added to the preparation. The additives may be added as needed in such an amount as not to damage the advantage of the present invention. Examples of the additives to be added to the preparation include excipient, disintegrant, binder and lubricant.

Examples of the excipient include crystalline cellulose, powdery cellulose, corn starch, potato starch, light anhydrous silicic acid, hydrous silicon dioxide, silicon dioxide, precipitated calcium carbonate, anhydrous dibasic calcium phosphate, magnesium oxide, calcium lactate, calcium silicate, magnesium aluminate metasilicate, synthetic hydrotalcite, synthetic aluminum silicate, sucrose fatty acid ester, hydrogenated oil, lactose, sucrose, D-mannitol, trehalose, glucose and fructose.
Examples of the disintegrant include carmellose, carmellose calcium, croscarmellose sodium, low-substituted hydroxypropyl cellulose, crospovidone, alginic acid, partially pregelatinized starch, and bentonite.
Examples of the binder include methyl cellulose, hydroxypropyl cellulose, hydroxypropylmethyl cellulose, polyvinyl alcohol, povidone, macrogol, gum arabic, sodium alginate, carboxyvinyl polymer, gelatin, dextrin, pectin, sodium polyacrylate and pullulan.
Examples of the lubricant include magnesium stearate, calcium stearate, talc, sucrose fatty acid ester, glycerin fatty acid ester, polyethylene glycol, and hydrogenated oil. These additives may be used either singly or in combination of two or more.

An injection may be prepared by adding a stabilizer, antiseptic and/or solubilizing agent to the preparation. It may be prepared as a solid preparation by filling a solution which may contain such an adjuvant in a container and lyophilizing the solution. Before use, it may be reconstituted. One dose may be packed in a container or a plurality of doses may be packed in the same container.

Examples of the preparation for external use include liquids and solutions, suspensions, emulsions, ointments, gels, creams, lotions and sprays.

The solid preparation may contain both an active compound and a pharmaceutically acceptable additive. It can be produced by mixing the active compound with additives such as filler, extender, binder, disintegrant, solubilization accelerator, humectant and lubricant selected as needed.

Examples of the liquid preparation include liquids and solutions, suspensions and emulsions. It may contain a suspending agent, emulsifier or the like as an additive.

The invention compound can be administered to animals, for example, by orally administering it as it or as a mixture in feed, or by preparing a solution of it and then orally administering it as is or as a mixture in drinking water or feed, or injecting the solution.

The preparation of the invention compound to be administered to animals can be prepared, by the technology ordinarily employed in this field, as powders, fine powders, soluble powders, syrups, liquids and solutions or injections as needed.

The pharmaceutical formulation examples will next be described.

| Formulation Example 1 [capsules] | |
|---|---|
| Compound of Example 1 | 100.0 mg |
| Corn starch | 23.0 mg |
| CMC calcium | 22.5 mg |
| Hydroxymethyl cellulose | 3.0 mg |
| Magnesium stearate | 1.5 mg |
| Total | 150.0 mg |

| Formulation Example 2 [Solution preparation] | |
|---|---|
| Compound of Example 1 | 1 to 10 g |
| Acetic acid or sodium hydroxide | 0.5 to 2 g |
| Ethyl para-hydroxybenzoate | 0.1 g |
| Purified water | 88.9 to 98.4 g |
| Total | 100.0 g |

| Formulation Example 3 [powders to be mixed with feed] | |
|---|---|
| Compound of Example 1 | 1 to 10 g |
| Corn starch | 98.5 to 89.5 g |
| Light anhydrous silicic acid | 0.5 g |
| Total | 100 g |

### EXAMPLES

The present invention will next be described by examples and reference examples. It should however be borne in mind that the present invention is not limited to or by them.

### Referential Example 1: 3-Difluoromethyl-N-[1-(R)-phenylethyl]pyrrolidin-2-one (optical isomer A, optical isomer B)

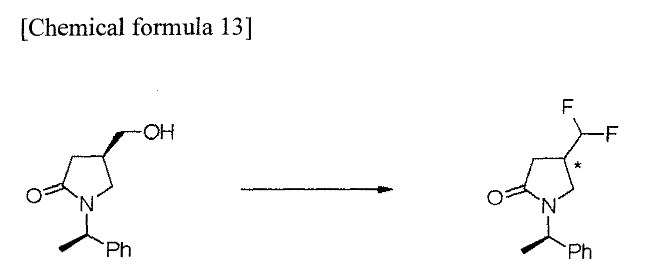

Dimethylsulfoxide (1.56 ml, 21.98 mmol) was added dropwise to a methylene chloride (40 ml) solution of oxalyl chloride (958 µl, 10.98 mmol) at -78°C and the resulting mixture was stirred at the same temperature for 30 minutes. A methylene chloride (10 ml) solution of 4-(S)-hydroxymethyl-N-[1-(R)-phenylethyl]pyrrolidin-2-one (2.19 g, 9.99 mmol) was added dropwise to the reaction mixture and the resulting mixture was stirred at the same temperature for 2 hours. Triethylamine (6.96 ml, 49.93 mmol) was then added, followed by stirring at room temperature for 1 hour. Water was added to the reaction mixture. The resulting mixture was extracted with methylene chloride. The extract was dried over sodium sulfate and then the solvent was removed under reduced pressure. The residue thus obtained was dissolved in methylene chloride (50 ml). After addition of diethylaminosulfur trifluoride (3.30 ml, 24.98 mmol) at -78°C, the temperature of the resulting mixture was raised gradually and stirring was continued at room temperature for 3 days. The reaction mixture was washed successively with a saturated aqueous solution of sodium bicarbonate and saturated brine, dried over sodium sulfate and distilled under reduced pressure to remove the solvent. The residue thus obtained was subjected to silica gel chromatography and eluted with an n-hexane:ethyl acetate = 2:1 to give 818 mg (34%) a lower polarity compound (optical isomer A) and then 795 mg (33%) of a higher polarity compound (optical isomer B) of the title compound.
Optical isomer A:
   ¹H-NMR (CDCl₃) δ: 1.55 (3H,2s), 2.46-2.72 (3H,m), 3.14 (1H,dd,J=8.30,10.30Hz), 3.35 (1H,dd,J=5.37,10.26Hz), 5.52 (1H,q,J=6.83Hz), 5.79 (1H,ddd,J=3.90,54.15,112.30Hz), 7.27-7.38 (5H,m).
Optical isomer B:
   ¹H-NMR (CDCl₃) δ: 1.54 (3H,2s), 2.46 (1H,dd,J=6.35,17.09Hz), 2.63 (1H,dd,J=9.63,17.09Hz), 2.72-2.81 (1H,m), 2.96 (1H,dd,J=5.37,10.74Hz), 3.44 (1H,dd,J=8.79,10.74Hz), 5.48-5.77 (2H,m), 7.26-7.37 (5H,m).

### Referential Example 2: trans-4-Difluoromethyl-3-hydroxy-N-[1-(R)-phenylethyl]-2-pyrrolidin-1-one (optical isomer A, optical isomer B)

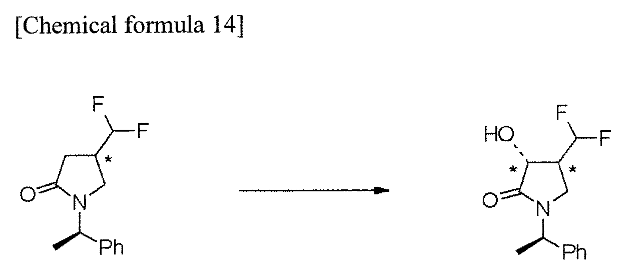

Under a nitrogen atmosphere, a 1.66 mol/l n-butyllithium hexane solution (1.82 ml) was added dropwise to a tetrahydrofuran (10 ml) solution of diisopropylamine (423 µl, 3.02 mmol) at -78°C and the resulting mixture was stirred at 0°C for 15 minutes. After cooling to -78°C, the reaction mixture was added dropwise to a tetrahydrofuran (5 ml) solution of 3-difluoromethyl-N-[1-(R)-phenylethyl]pyrrolidin-2-one (optical isomer A; 555 mg, 2.32 mmol) at -78°C under a nitrogen atmosphere. The reaction mixture was stirred at the same temperature for 1 hour, followed by stirring at the same temperature for 1 hour under an oxygen atmosphere. After completion of the reaction, a 5% aqueous solution of sodium thiosulfate was added to the reaction mixture and tetrahydrofuran was removed under reduced pressure. The residue was extracted with chloroform. The combined organic layers were dried over sodium sulfate and the solvent was removed under reduced pressure. The residue was then subjected to silica gel chromatography and eluted with a 3% methanol-chloroform to give 357 mg (60%) of the title compound (optical isomer A).

In a similar reaction and operation using 3-difluoromethyl-N-[1-(R)-phenylethyl]pyrrolidin-2-one (optical isomer B; 555 mg, 2.32 mmol), 411 mg (69%) of the title compound (optical isomer B) was obtained.
Optical isomer A:
   ¹H-NMR (CDCl₃) δ: 1.55 (3H,2s), 2.56-2.66 (1H,m), 3.07 (1H,dd,J=8.79,10.25Hz), 3.30-3.33 (1H,m), 3.50 (1H,brs), 4.39 (1H,d,J=8.8Hz), 5.48 (1H,q,J=6.84Hz), 5.90 (1H,ddd,J=3.90,52.24,108.39Hz), 7.27-7.38 (5H,m).
Optical isomer B:
   ¹H-NMR (CDCl₃) δ:1.58 (3H,2s), 2.71-2.80 (1H,m), 2.93 (1H,t,J=10.79Hz), 3.40 (1H,dd,J=8.81,10.26Hz), 3.89 (1H,brs), 4.34 (1H,d,J=8.79Hz), 5.47 (1H,q,J=7.33Hz), 6.00 (1H,J=ddd,3.41,56.15,111.32Hz), 7.26-7.38 (5H,m).

### Referential Example 3: cis-3-Azido-4-fluoromethyl-N-[1-(R)-phenylethyl]-2-pyrrolidin-2-one (optical isomer A, optical isomer B)

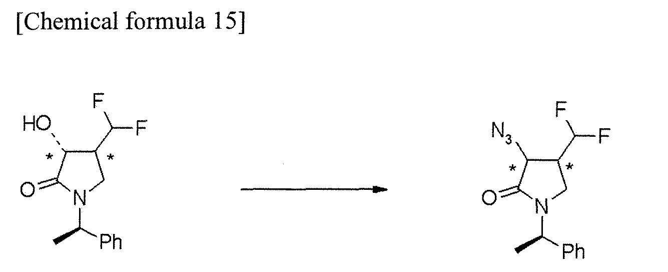

Triethylamine (1.42 ml, 10.19 mmol) was added to a methylene chloride (20 ml) solution of trans-4-difluoromethyl-3-hydroxy-N-[1-(R)-phenylethyl]-2-pyrolidin-2-one (optical isomer A; 1.30 g, 5.09 mmol). Under ice cooling, methanesulfonyl chloride (591 µl, 7.64 mmol) was added dropwise and the resulting mixture was stirred at the same temperature for 30 minutes. After completion of the reaction, the reaction mixture was washed with a 10% aqueous solution of citric acid, dried over sodium sulfate and the solvent was removed under reduced pressure. The residue thus obtained was dissolved in N,N-dimethylformamide (40 ml). Sodium azide (1.32 g, 20.3 mmol) was added thereto, and the resulting mixture was stirred at 100°C for 3 hours. Water was added to the reaction mixture. The resulting mixture was extracted with ethyl acetate. The combined organic layers were dried over sodium sulfate and the solvent was removed under reduced pressure. The residue thus obtained was subjected to silica gel chromatography and eluted with ethyl acetate:hexane=1:4 to give 563 mg (39%) of the title compound (optical isomer A).

In a similar reaction and operation using trans-4-difluoromethyl-3-hydroxy-N-[1-(R)-phenylethyl]-2-pyrrolidin-2-one (optical isomer B: 1.00 g, 3.91 mmol), 433 mg (39%) of the title compound (optical isomer B) was obtained.
Optical isomer A:
   ¹H-NMR (CDCl₃) δ: 1.58 (3H,2s), 2.68-2.75 (1H,m), 3.00 (1H,dd,J=7.81,10.74Hz), 3.41 (1H,dd,J=5.37,10.25Hz), 4.30 (1H,d,J=8.30Hz), 5.48 (1H,q,J=7.33Hz), 5.99 (1H,ddd,J=3.89,55.67,110.84Hz), 7.26-7.38 (5H,m).
Optical isomer B:
   ¹H-NMR (CDCl₃) δ:1.56 (3H,2s), 2.75-2.85 (1H,m), 3.00 (1H,dd,J=5.86,10.25Hz), 3.32 (1H,dd,J=7.81,10.25Hz), 4.31 (1H,d,J=8.30Hz), 5.49 (1H,q,J=6.84Hz), 5.86 (1H,J=ddd,5.37,56.66,110.84Hz), 7.26-7.39 (5H,m).

### Referential Example 4: cis-3-(Tert-butoxycarbonylamino)-4-difluoromethyl-N-[1-(R)-phenylethyl]pyrrolidine (optical isomer A)

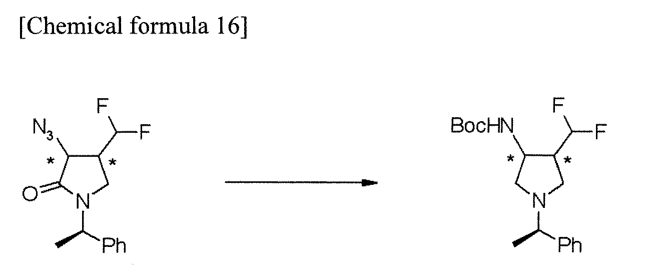

Lithium aluminum hydride (762 mg, 20.08 mmol) was added in portions to a tetrahydrofuran (50 ml) solution of cis-3-azido-4-fluoromethyl-N-[1-(R)-phenylethyl]-2-pyrrolidin-2-one (optical isomer A; 563 mg, 2.00 mmol) under ice cooling, followed by heating under reflux for 1.5 hours. Under ice cooling, water (762 µl), a 15% aqueous solution (762 µl) of sodium hydroxide and water (762 µl) were successively added dropwise to the reaction mixture and the resulting mixture was stirred overnight at room temperature. After the insoluble material was filtered off using celite, the solvent was removed under reduced pressure. The residue was dissolved in tetrahydrofuran (50 ml). To the resulting solution was added di(tertiary butyl) dicarbonate (873 mg, 4.00 mmol). The resulting mixture was stirred at room temperature for 3 hours. After the solvent was removed under reduced pressure, the residue was subjected to silica gel chromatography and eluted with ethyl acetate:hexane=1:4 to give 439 mg (64%) of the title compound (optical isomer A).
Optical isomer A:
   ¹H-NMR (CDCl₃) δ:1.43 (3H,s), 1.56 (9H,s), 2.63-2.78 (5H,m), 3.24-3.29 (1H,m), 4.38-4.46 (1H,m), 4.84 (1H,d,J=9.28Hz), 5.70-5.98 (1H,m), 7.26-7.37 (5H,m).

### Referential Example 5: cis-3-(Tert-butoxycarbonylamino)-4-difluoromethyl-N-[1-(R)-phenylethyl]pyrrolidine (optical isomer B)

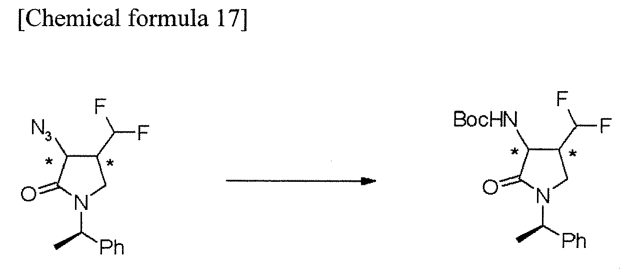

Di(tert-butyl)dicarbonate (1.30 g, 5.96 mmol) and 10% palladium carbon (800 mg) were added to an ethanol (20 ml) solution of cis-3-(tert-butoxycarbonylamino)-4-difluoromethyl-N-[1-(R)-phenylethyl]pyrrolidine (optical isomer B; 835 mg, 2.98 mmol). The resulting mixture was subjected to catalytic hydrogenation overnight at room temperature. After the catalyst was filtered off, the solvent was removed under reduced pressure. The resulting residue was subjected to silica gel chromatography and eluted with ethyl acetate:hexane=1:2 to give 764 mg (72%) of the title compound (optical isomer B).
Optical isomer B:
   ¹H-NMR (CDCl₃) δ: 1.45 (9H,s), 1.55 (3H,2s), 2.97-3.14 (1H,m), 3.22 (1H,d,J=10.75Hz), 3.36 (1H,dd,J=7.33,10.74Hz), 4.43-4.52 (1H,m), 5.13 (1H,brs), 5.48 (1H,q,J=7.32Hz), 5.61-5.89 (1H,m), 7.26-7.36 (5H,m).

### Referential Example 6: cis-3-(Tert-butoxycarbonylamino)-4-difluoromethyl-N-[1-(R)-phenylethyl]pyrrolidine (optical isomer B)

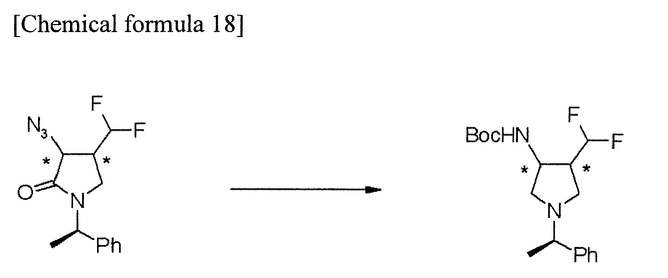

A 1 mol tetrahydrofuran (6.47 ml) solution of borane-tetrahydrofuran complex was added dropwise to a tetrahydrofuran (20 ml) solution of cis-3-(tert-butoxycarbonylamino)-4-difluoromethyl-N-[1-(R)-phenylethyl]pyrrolidine (optical isomer B; 764 mg, 2.16 mmol) under ice cooling. The resulting mixture was stirred overnight at room temperature. After the solvent was removed under reduced pressure, a mixed solvent (40 ml) of ethanol-water (4: 1) was added to the residue. In the presence of triethylamine (8 ml), the resulting mixture was heated under reflux for 2 hours. After cooling, the solvent was removed under reduced pressure. To the residue thus obtained was added chloroform. The resulting mixture was washed with saturated brine. The organic layer was dried over sodium sulfate and then the solvent was removed under reduced pressure. The residue thus obtained was subjected to silica gel chromatography and eluted with ethyl acetate:hexane=1:4 to give 519 mg (71 %) of the title compound (optical isomer B).
Optical isomer B:
   ¹H-NMR (CDCl₃) δ:1.36 (3H,2s), 1.43 (9H,s), 2.29-2.33 (1H,m), 2.53-2.90 (4H,m), 3.26 (1H,q,J=6.35Hz), 4.32-4.41 (1H,m), 4.82 (1H,d,J=8.79Hz), 5.74-6.02 (1H,m), 7.22-7.33 (5H,m).

### Referential Example 7: cis-3-(Tert-butoxycarbonylamino)-4-difluoromethylpyrrolidine (optical isomer A, optical isomer B)

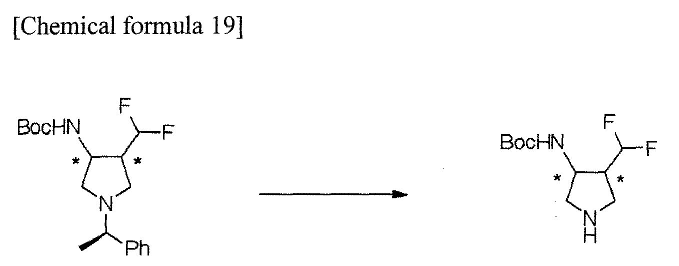

To an ethanol (40 ml) solution of cis-3-(tert-butoxycarbonylamino)-4-difluoromethyl-N-[1-(R)-phenylethyl]pyrrolidine (optical isomer A: 439 mg, 1.29 mmol) was added 10% palladium carbon (440 mg). The resulting mixture was subjected to catalytic hydrogenation overnight at 50°C. After the catalyst was filtered off, the solvent was removed under reduced pressure, whereby a crude title compound was obtained in a quantitative yield. The resulting compound was provided for the subsequent reaction without purification.
In a similar reaction and operation using cis-3-(tert-butoxycarbonylamino)-4-difluoromethyl-N-[1-(R)-phenylethyl]pyrrolidine (optical isomer B; 400 mg, 1.18 mmol), a crude title compound (optical isomer B) was obtained (quant.).

### Example 1: 10-[3-(S)-Amino-4-(S)-fluoromethylpyrrolidin-1-yl]-9-fluoro-2,3-dihydro-3-(S)-methyl-7-oxo-7H-pyrido[1,2,3-de][1,4]benzoxazine-6-carboxylic acid

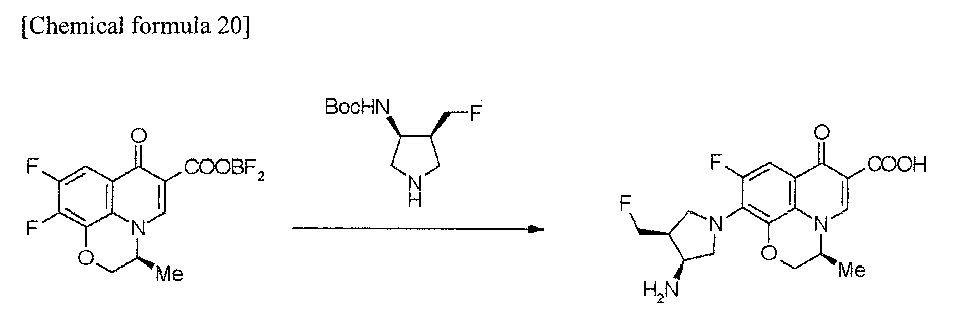

To a dimethylsulfoxide (4 ml) solution of 9,10-difluoro-2,3-dihydro-3(S)-methyl-7-oxo-7H-pyrido[1,2,3-de][1,4]benzoxazine-6-carboxylic acid BF₂ chelate (328 mg, 1.00 mmol) were added 3(S)-(tert-butoxycarbonylamino)-4-(S)-fluoromethylpyrrolidine (316 mg, 1.44 mmol) and triethylamine (300 µl). The resulting mixture was stirred at room temperature for 1 day.
After triethylamine was removed under reduced pressure, a 10% aqueous solution of citric acid was added to the residue. The precipitate thus formed was collected by filtration, washed with water and then dissolved in 80% aqueous ethanol (50 ml). Triethylamine (5 ml) was added to the resulting solution, followed by heating under reflux overnight. After the solvent was removed under reduced pressure, concentrated hydrochloric acid was added to the residue and the mixture was stirred at room temperature for 15 minutes. The reaction mixture was washed with chloroform and then alkalified with a 50% aqueous solution of sodium hydroxide under ice cooling. The resulting alkali solution was then adjusted to pH 7.4 with concentrated hydrochloric acid. The aqueous layer was extracted with chloroform (300 ml x 3). The organic layer was dried over sodium sulfate and the solvent was removed under reduced pressure. The residue was purified by recrystallization from 28% aqueous ammonia/ethanol to give 189 mg (50%) of the title compound. mp:243-245°C.
¹H-NMR (0.1NNaOD)δ:1.49 (3H,d,J=6.84Hz), 2.68-2.74 (1H,m), 3.39-3.41 (1H,m), 3.65-3.68 (3H,m), 3.60-3.65 (1H,m), 3.92-3.95 (1H,m), 4.29-4.32 (1H,m), 4.46-4.49 (1H,m), 4.57-5.59 (1H.m), 7.50 (1H,d,J=14.16Hz), 8.31 (1H,s).
Anal.: Calcd for C₁₈H₁₉F₂N₃O₄·0.25H₂O C,56.32; H,5.12; N,10.95.
Found C,56.65; H,4.93; N,10.87.

### Example 2: 10-[cis-3-Amino-4-difluoromethylpyrrolidin-1-yl]-9-fluoro-2,3-dihydro-3-(S)-methyl-7-oxo-7H-pyrido[1,2,3-de][1,4]benzoxazine-6-carboxylic acid (optical isomer A)

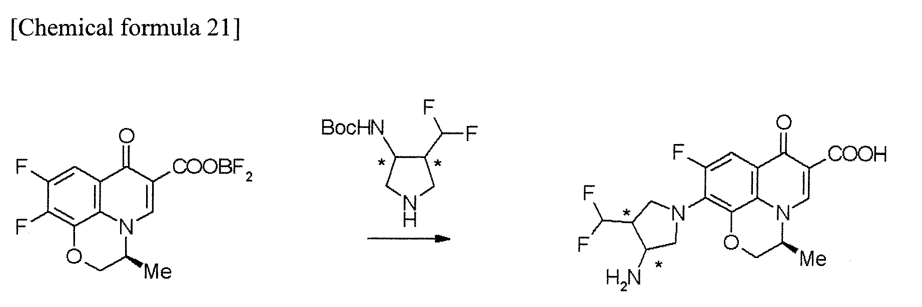

To a dimethylsulfoxide (3 ml) solution of 9,10-difluoro-2,3-dihydro-3(S)-methyl-7-oxo-7H-pyrido[1,2,3-de][1,4]benzoxazine-6-carboxylic acid BF₂ chelate (221 mg, 0.67 mmol) were added cis-3-(tert-butoxycarbonylamino)-4-difluoromethylpyrrolidine (optical isomer A; 159 mg, 0.67 mmol) and triethylamine (300 µl). The resulting mixture was stirred overnight at room temperature and then at 40°C for 1 day. After triethylamine was removed under reduced pressure, a 10% aqueous solution of citric acid was added. The precipitate thus formed was collected by filtration, washed with water and then dissolved in 80% aqueous ethanol (20 ml). Triethylamine (2 ml) was added to the resulting solution, followed by heating under reflux for 2 hours. After the solvent was removed under reduced pressure, concentrated hydrochloric acid (5 ml) was added to the residue and the mixture was stirred at room temperature for 15 minutes. The reaction mixture was washed with chloroform and then alkalified with a 50% aqueous solution of sodium hydroxide under ice cooling. The resulting alkali solution was then adjusted to pH 7.4 with concentrated hydrochloric acid. The aqueous layer was extracted with chloroform (300 ml x 3). The organic layer was dried over sodium sulfate and the solvent was removed under reduced pressure. The residue was purified by recrystallization from 28% aqueous ammonia/ethanol to give 127 mg (48%) of the title compound (optical isomer A). mp:247-252°C.
¹H-NMR (0.1NNaOD) δ: 1.50 (3H,d,J=5.86Hz), 2.73-2.84 (1H,m), 3.40-3.43 (1H,m), 3.62-3.68 (2H,m), 3.60-3.75 (1H,m), 3.88-3.91 (2H,m), 4.29-4.31 (1H,m), 4.45-4.48 (1H,m), 4.55-4.59 (1H,m), 6.18 (1H,ddd,J=5.86,55.66,116.18Hz), 7.48 (1H,d,J=14.16Hz), 8.31 (1H,s).
Anal.: Calcd. for C₁₈H₁₈F₃N₃O₄·0.25H₂O C,53.80; H,4.64; N,10.46.
Found C,54.80; H,4.56; N,10.41.

### Example 3: 10-[cis-3-Amino-4-difluoromethylpyrrolidin-1-yl]-9-fluoro-2,3-dihydro-3-(S)-methyl-7-oxo-7H-pyrido[1,2,3-de][1,4]benzoxazine-6-carboxylic acid (optical isomer B)

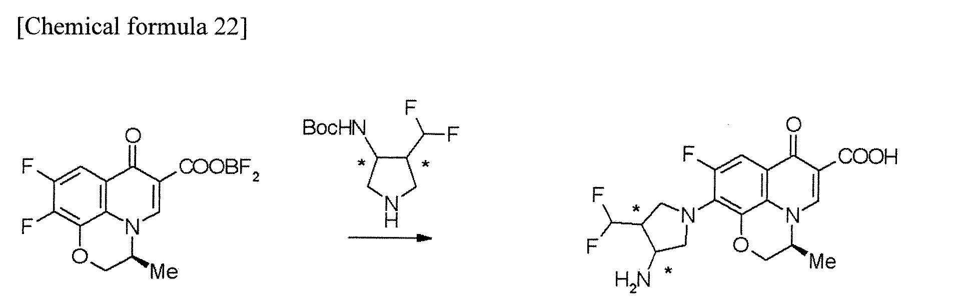

To a dimethylsulfoxide (4 ml) solution of 9,10-difluoro-2,3-dihydro-3-(S)-methyl-7-oxo-7H-pyrido[1,2,3-de][1,4]benzoxazine-6-carboxylic acid BF₂ chelate (292 mg, 0.89 mmol) were added cis-3-(tert-butoxycarbonylamino)-4-difluoromethylpyrrolidine (optical isomer B; 210 mg, 0.89 mmol) and triethylamine (400 µl). The resulting mixture was stirred overnight at room temperature and at 40°C for one day. After triethylamine was removed under reduced pressure, a 10% aqueous solution of citric acid was added. The precipitate thus formed was collected by filtration, washed with water and then dissolved in 80% aqueous ethanol (20 ml). Triethylamine (2 ml) was added to the resulting solution, followed by heating under reflux for 2 hours. After the solvent was removed under reduced pressure, concentrated hydrochloric acid (5 ml) was added to the residue and the mixture was stirred at room temperature for 15 minutes. The reaction mixture was washed with chloroform and then alkalified with a 50% aqueous solution of sodium hydroxide under ice cooling. The resulting alkali solution was then adjusted to pH 7.4 with concentrated hydrochloric acid. The aqueous layer was extracted with chloroform (300 ml × 3). The organic layer was dried over sodium sulfate and the solvent was removed under reduced pressure. The residue was purified by recrystallization from 28% aqueous ammonia/ethanol to give 226 mg (64%) of the title compound (optical isomer B).
mp:264-268°C.
¹H-NMR (0.1NNaOD) δ: 1.49 (3H,d,J=6.84Hz), 2.73-2.88 (1H,m), 3.38-3.41 (1H,m), 3.61-3.66 (1H,m), 3.73-3.77 (1H,m), 3.90-3.95 (2H,m), 4.31-4.34 (1H,m), 4.46-4.49 (1H,m), 4.57-4.58 (1H.m), 6.18 (1H,ddd,J=5.35,55.67,111.82Hz), 7.48 (1H,dd,J=2.44,14.16Hz), 8.31 (1H,s).
Anal.: Calcd. for C₁₈H₁₈ F₃ N₃ O₄ C,54.41; H,4.57; N,10.58.
Found C,54.18; H,4.49; N,10.57.

### Test 1:

The antimicrobial activity of the compounds of the present invention is measured in accordance with the standard method designated by Japanese Society of Chemotherapy and MIC (microgram/ml) values are shown as its results in Table 1. The MIC values of levofloxacin (LVFX), ciprofloxacin (CPFX) and comparative compound (the below-described compound obtained in Example 2 of Patent Document 2) are also shown for comparison with that of the invention compounds.

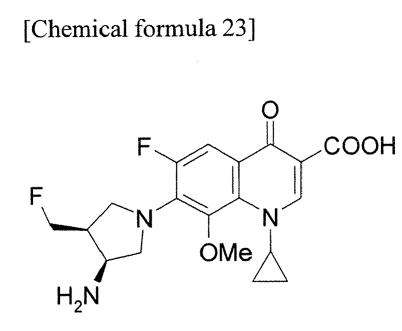

### Comparative compound

The results have revealed that compared with the comparative compound, the compounds of the above-described examples have a broad spectrum and potent antimicrobial activity against both ram positive and negative bacteria including resistant bacteria. In particular, it has been found that they have potent antimicrobial activity against gram positive bacteria such as methicillin-resistant *Staphylococcus aureus* (MRSA) and penicillin-resistant *Streptococcus pneumoniae* (PRSP).

**[Table 1]**

| | Ex. 1 | Ex. 2 | Ex.3 | LVFX | CPFX | Comparative Compound |
|---|---|---|---|---|---|---|
| *E. coli, NIHJ* | 0.006 | ≤0.003 | 0.012 | 0.12 | ≤0.003 | ≤0.003 |
| *S. flexneri,* 2A 5503 | 0.012 | 0.012 | 0.025 | 0.025 | 0.006 | 0.006 |
| *Pr. vulgalis,* 08601 | 0.012 | 0.012 | 0.025 | 0.012 | ≤0.003 | 0.006 |
| *K.pneumoniae,* Type I | 0.05 | 0.05 | 0.1 | 0.1 | 0.025 | 0.025 |
| *Ser. marcescens,* 10100 | 0.05 | 0.05 | 0.1 | 0.1 | 0.025 | 0.05 |
| *Ps. aeruginosa,* 32104 | 0.1 | 0.2 | 0.39 | 0.2 | 0.005 | 0.2 |
| *Ps. aeruginosa,* 32121 | 0.025 | 0.05 | 0.1 | 0.1 | 0.025 | 0.05 |
| *S. maltophilia,* IID-1275 | 0.39 | 0.2 | 0.39 | 0.39 | 0.78 | 0.05 |
| *S. aureus,* 209P | 0.025 | 0.025 | 0.05 | 0.2 | 0.1 | 0.012 |
| *S. epidermidis,* 56500 | 0.1 | 0.1 | 0.2 | 0.39 | 0.2 | 0.05 |
| *Str. pyogenes,* G-36 | 0.2 | 0.2 | 0.78 | 0.2 | 1.56 | 0.1 |
| *Str. faecalis,* ATCC-19433 | 0.2 | 0.39 | 0.78 | 0.78 | 0.78 | 0.1 |
| *S. aureus,* 870307 | 1.56 | 1.56 | 3.13 | >6.25 | 3.13 | 0.39 |
| *Str. pneumoniae,* J24 | 0.1 | 0.2 | 0.39 | 0.78 | 0.1 | 0.025 |

### Test 2:

The single intravenous dosing toxicity test in mice using the invention compound was carried out in the following manner. The compound was dissolved in and diluted with 0.1 mol/l NaOH/physiological saline. After the diluted solution was sterilized by filtration through a 0.22 µm filter of Millex GS, the resulting sterilized solution was intravenously administered to six-week-old Slc:ddY male mice at an administration rate of 10 ml/kg or 0.2 ml/min as a single dose. The results are shown in Table 2. Similar to Test 1, the compound obtained in Example 2 of Patent Document 2 was employed as a comparative compound.
The results have revealed that three of five mice died by the administration of the comparative compound, while any one of five mice did not die when the compounds obtained in Examples of the present invention were administered. This suggests that the compounds of the present invention have week acute toxicity.

**[Table 2]**

| | Example 1 | Comparative Compound |
|---|---|---|
| Dose mg/kg | Number of deaths/total number | Number of deaths/total number |
| 150 | 0/5 | 3/5 |

The present invention was so far described specifically referring to specific embodiments. It is obvious to those skilled in the art that various changes or modifications may be made without departing from the spirit and scope of the invention.
The present application is based on Japanese patent application (Japanese Patent Application No. 2004-25982) filed on February 2, 2004, the content of which is hereby incorporated by reference into this application.

### INDUSTRIAL APPLICABILITY

The compounds of the present invention have a broad spectrum and excellent antimicrobial activity against both gram negative bacteria and gram positive bacteria.
In particular, they exhibit potent antimicrobial activity against resistant gram positive bacteria such as methicillin-resistant *Staphylococcus aureus* (MRSA) and penicillin resistant *Streptococcus pneumoniae* (PRSP) and also quinolone resistant bacteria so that they are useful as an antimicrobial compound to be used for chemotherapy for bacterial infections.

## Claims

1. A compound represented by the following formula (1): 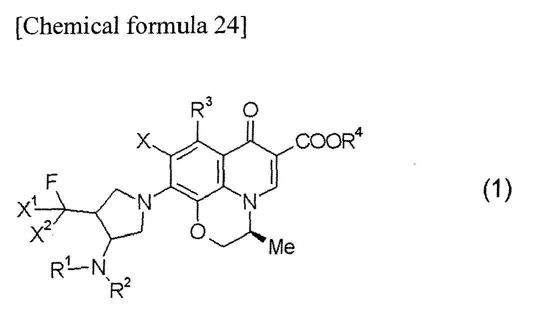 [wherein, R¹ represents a hydrogen atom, an alkyl group having from 1 to 6 carbon atoms or a cycloalkyl group having from 3 to 6 carbon atoms, or a substituted carbonyl group derived from an amino acid, dipeptide or tripeptide, with the proviso that the alkyl group may have, as the substituent, at least one group selected from the group consisting of a hydroxyl group, an amino group, halogen atoms, alkylthio groups having from 1 to 6 carbon atoms, and alkoxy groups having from 1 to 6 carbon atoms;
R² represents a hydrogen atom, an alkyl group having from 1 to 6 carbon atoms or a cycloalkyl group having from 3 to 6 carbon atoms, with the proviso that the alkyl group may have, as the substituent, at least one group selected from the group consisting of a hydroxyl group, an amino group, halogen atoms, alkylthio groups having from 1 to 6 carbon atoms, and alkoxy groups having from 1 to 6 carbon atoms;
R³ represents a hydrogen atom, amino group, halogen atom or alkyl group having from 1 to 6 carbon atoms, with the proviso that the amino group may have, as the substituent, one or two groups selected from the group consisting of a formyl group, alkyl groups having from 1 to 6 carbon atoms and acyl groups having from 2 to 6 carbon atoms;
R⁴ represents a hydrogen atom, phenyl group, acetoxymethyl group, pivaloyloxymethyl group, ethoxycarbonyl group, choline group, dimethylaminoethyl group, 5-indanyl group, phthalidinyl group, 5-alkyl-2-oxo-1,3-dioxol-4-ylmethyl group, 3-acetoxy-2-oxobutyl group, alkyl group having from 1 to 6 carbon atoms, alkoxymethyl group having from 2 to 7 carbon atoms, or a phenylakyl group composed of an alkylene group having from 1 to 6 carbon atoms and a phenyl group;
X¹ and X² each independently represents a hydrogen atom or halogen atom, and
X represents a hydrogen atom or a halogen atom], salt thereof, or a hydrate thereof.

2. A compound, salt thereof, or a hydrate thereof according to Claim 1, wherein the compound of the formula (1) has a structure represented by the following formula: 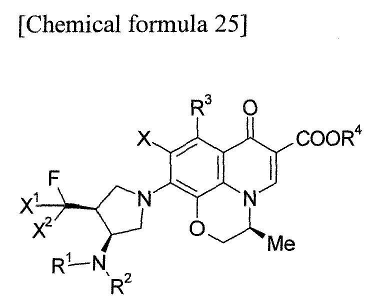 [wherein, R¹, R², R³, R⁴, X¹, X² and X have the same meanings as described above].

3. A compound, salt thereof, or a hydrate thereof according to Claim 1 or 2, wherein in the formula (1), X¹ and X² each represents a hydrogen atom.

4. A compound, salt thereof, or a hydrate thereof according to Claim 1 or 2, wherein in the formula (1), either one of X¹ or X² represents a fluorine atom, while the other one represents a hydrogen atom.

5. A compound, salt thereof, or a hydrate thereof according to any one of Claims 1 to 4, wherein in the formula (1), R¹ and R² each represents a hydrogen atom.

6. A compound, salt thereof, or a hydrate thereof according to any one of Claims 1 to 4, wherein in the formula (1), R¹ represents a substituted carbonyl group derived from an amino acid, dipeptide or tripeptide and R² represents a hydrogen atom.

7. A compound, salt thereof, or a hydrate thereof according to any one of Claims 1 to 4, wherein in the formula (1), either one of R¹ or R² represents a hydrogen atom, while the other one is a cyclopropyl group.

8. A compound, salt thereof, or a hydrate thereof according to any one of Claims 1 to 7, wherein in the formula (1), wherein X represents a fluorine atom.

9. A compound, salt thereof, or a hydrate thereof according to any one of Claims 1 to 8, wherein in the formula (1), R³ represents a hydrogen atom.

10. A compound, salt thereof, or a hydrate thereof according to any one of Claims 1 to 9, wherein in the formula (1), R⁴ represents a hydrogen atom.

11. 10-[3-Amino-4-fluoromethylpyrrolidin-1-yl]-9-fluoro-2,3-dihydro-3-(S)-methyl-7-oxo-7H-pyrido[1,2,3-de][1,4]benzoxazine-6-carboxylic acid, salt thereof, or a hydrate thereof.

12. 10-[3-Amino-4-difluoromethylpyrrolidin-1-yl]-9-fluoro-2,3-dihydro-3-(S)-methyl-7-oxo-7H-pyrido[1,2,3-de][1,4]benzoxazine-6-carboxylic acid, salt thereof, or a hydrate thereof.

13. 10-[cis-3-Amino-4-fluoromethylpyrrolidin-1-yl]-9-fluoro-2,3-dihydro-3-(S)-methyl-7-oxo-7H-pyrido[1,2,3-de][1,4]benzoxazine-6-carboxylic acid, salt thereof, or a hydrate thereof.

14. 10-[cis-3-Amino-4-difluoromethylpyrrolidin-1-yl]-9-fluoro-2,3-dihydro-3-(S)-methyl-7-oxo-7H-pyrido[1,2,3-de][1,4]benzoxazine-6-carboxylic acid, salt thereof, or a hydrate thereof.

15. A compound or salt thereof, or a hydrate thereof according to any one of Claims 1 to 14, wherein the compound of the formula (1) is a stereochemically pure compound.

16. 10-[3-(S)-Amino-4-(S)-fluoromethylpyrrolidin-1-yl]-9-fluoro-2,3-dihydro-3-(S)-methyl-7-oxo-7H-pyrido[1,2,3-de][1,4]benzoxazine-6-carboxylic acid, salt thereof, or a hydrate thereof.

17. 10-[3-(S)-Amino-4-(S)-difluoromethylpyrrolidin-1-yl]-9-fluoro-2,3-dihydro-3-(S)-methyl-7-oxo-7H-pyrido[1,2,3-de][1,4]benzoxazine-6-carboxylic acid, salt thereof, or a hydrate thereof.

18. A medicament comprising as an effective ingredient a compound, salt thereof, or a hydrate thereof as claimed in any one of Claims 1 to 17.

19. An antimicrobial agent comprising as an effective ingredient a compound, salt thereof, or a hydrate thereof as claimed in any one of Claims 1 to 17.

20. A therapeutic agent for infections, which comprises as an effective ingredient a compound, salt thereof, or a hydrate thereof as claimed in any one of Claims 1 to 17.

21. A method of treating a disease, which comprises administering an effective amount of a compound, salt thereof, or a hydrate thereof as claimed in any one of Claims 1 to 17.

22. A method of treating an infection, which comprises administering an effective amount of a compound, salt thereof, or a hydrate thereof as claimed in any one of Claims 1 to 17.

23. A process of producing a medicament, which comprises incorporating a compound, salt thereof, or a hydrate thereof as claimed in any one of Claims 1 to 17 as an effective ingredient.

24. A process of producing an antimicrobial agent, which comprises incorporating a compound, salt thereof, or a hydrate thereof as claimed in any one of Claims 1 to 17 as an effective ingredient.

25. A process of producing a therapeutic agent for infections, which comprises incorporating a compound, salt thereof, or a hydrate thereof as claimed in any one of Claims 1 to 17 as an effective ingredient.

26. A compound represented by the following formula (3): 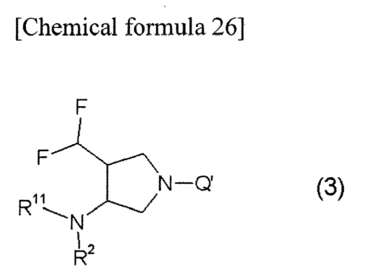 [wherein, R¹¹ represents a hydrogen atom, an alkyl group having from 1 to 6 carbon atoms or a cycloalkyl group having from 3 to 6 carbon atoms, or an amino-protecting group, or a substituted carbonyl group derived from an amino acid, dipeptide or peptide having an amino group which is unsubstituted or may be protected, with the proviso that the alkyl group may have, as the substituent, at least one group selected from the group consisting of a hydroxyl group, an amino group, halogen atoms, alkylthio groups having from 1 to 6 carbon atoms and alkoxy groups having from 1 to 6 carbon atoms;
R² has the same meaning as defined in the formula (1); and
Q' means an amino-protecting group or hydrogen atom], salt thereof, or a hydrate thereof.

27. A compound, salt thereof, or a hydrate thereof according to Claim 1, wherein the compound of the formula (3) is a compound having a structure represented by the following formula (4): 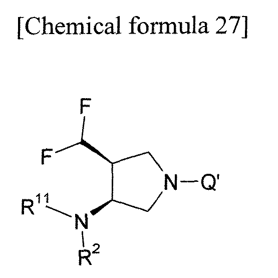 [wherein, R¹¹, R² and Q' have the same meanings as defined above].

28. A compound, salt thereof, or a hydrate thereof according to Claim 26 or 27, wherein the amino protecting group is selected from the group consisting of alkoxycarbonyl groups which may have a substituent, aralkyloxycarbonyl groups which may have a substituent, acyl groups which may have a substituent, alkyl groups which may have a substituent, aralkyl groups which may have a substituent, and substituted silyl groups.

29. A compound, salt thereof, or a hydrate thereof according to Claim 26, 27 or 28, wherein the amino protecting group is selected from the group consisting of; tertiary butoxycarbonyl group, 2,2,2-trichloroethoxycarbonyl group, benzyloxycarbonyl group, para-methoxybenzyloxycarbonyl group, para-nitrobenzyloxycabonyl group, acetyl group, methoxyacetyl group, trifluoroacetyl group, chloroacetyl group, pivaloyl group, formyl group, benzoyl group, tertiary butyl group, benzyl group, paranitrobenzyl group, para-methoxybenzyl group, (R)-1-phenylethyl group, (S)-1-phenylethyl group, triphenylmethyl group, methoxymethyl group, tertiary butoxymethyl group, tetrahydropyranyl group, 2,2,2-trichloroethoxymethyl group, trimethylsilyl group, isopropyldimethylsilyl group, tertiary butyldimethylsilyl group, tribenzylsilyl group, and tertiary butyldiphenylsilyl group.

30. A compound, salt thereof, or a hydrate thereof according to any one of Claims 26 to 29, wherein R¹ and Q' represent amino protecting groups which are not the same.

31. A compound, salt thereof, or a hydrate thereof according to any one of Claims 26 to 29, wherein R¹¹ and Q' represent amino protecting groups which are to be deprotected or cleaved under different reaction conditions.

32. Cis-3-(tert-butoxycarbonylamino-4-difluoromethyl)-1-(R)-phenylmethylpyrrolidine, salt thereof, or a hydrate thereof

33. Cis-3-(tert-butoxycarbonylamino-4-difluoromethyl)-pyrrolidine, salt thereof, or a hydrate thereof.

34. A compound, salt thereof, or a hydrate thereof according to any one of Claims 26 to 33, wherein the compound of the formula (3) is a stereochemically single compound.

35. 3-(S)-(Tert-butoxycarbonylamino)-4-(S)-difluoromethyl-1-(R)-phenylmethylpyrrolidine, salt thereof, or a hydrate thereof

36. 3-(S)-(Tert-butoxycarbonylamino)-4-(S)-difluoromethyl-pyrrolidine, salt thereof, or a hydrate thereof.

37. Use of a compound, salt thereof, or a hydrate thereof as claimed in any one of Claims 26 to 36 for the production of the compound represented by the formula (1).
